# EUROPEAN PATENT APPLICATION

(11) **EP 1 881 326 A1**
(43) Date of publication of application: **23.01.2008**
(21) Application number: 06291163.1
(22) Date of filing: 18.07.2006
(51) Int. Cl.: G01N 33/569, G01N 33/68, G01N 33/574

(54) **Myeloid progenitor cells for diagnosis of central nervous system diseases**

(71) Applicant: INSERM (Institut National de la Santé et de la Recherche Médicale), 75654 Paris Cedex 13 (FR)
(72) Inventor: Nataf, Serge, 69003 Lyon (FR); Davoust, Nathalie, 69003 Lyon (FR); Belin, Marie-Françoise, 69002 Lyon (FR); Lachuer, Joël, 01600 Trévoux (FR)
(74) Representative: Chajmowicz, Marion

(57) **Abstract**

The invention relates to a method for diagnosis or prognosis of a disease of the central nervous system, which method comprises detecting and/or quantifying CD34+ CD14+ or CD34+ CD11b+ myeloid progenitor cells in a blood sample of a test subject.

## Description

The invention relates to the identification of a subset of cells in blood of a patient afflicted with a disease of the central nervous system, and to the diagnostic uses of said cells.

Although the ontogeny of microglial cells has long been unclear, microglia are now recognized as the resident macrophages of the central nervous system (CNS). As such, microglia has been involved in the pathophysiology of numerous inflammatory or non-inflammatory CNS disorders such as multiple sclerosis, Alzheimer's disease and amyotrophic lateral sclerosis. The constant renewal of microglia by blood-circulating cells is currently accepted and bone marrow (BM) transfer experiments have allowed the haematopoietic origin of microglial cells to be formally established. Moreover, an accelerated turnover of microglia by blood-circulating BM-derived cells was shown to occur under several neuroinflammatory conditions, including experimental allergic encephalomyelitis (EAE). However, the exact identity of these blood cells and the different steps allowing them to migrate and differentiate into microglia have been unknown so far in humans.

In mice, bone marrow CD34+ B220+ progenitors target the inflamed brain and display in vitro differentiation potential toward microglia (Davoust et al, 2004, Fifth congress of the International Society of Neuro-Immunology, Venize, 2004)

Other studies showed that blood-circulating CD34+ CD14+ cells in humans are endowed with a high proliferative potential, are able to cross the vascular endothelium and display differentiation potential toward macrophages or dendritic cells (Ferrero et al, 1998; Tjonnfjord et al, 1996). Also, glioma cells were recently shown to direct the migration of exogenously-delivered CD34+ progenitors from blood to brain (Tabatabai et al 2005).

### Summary of the invention:

The inventors have now discovered that CD34+ CD14+ or CD34+ CD11 b+ myeloid progenitor cells accumulate in the blood of patient afflicted with a disease of the central nervous system (CNS). The inventors have also discovered that such myeloid progenitors accumulate in the blood of mice, in an experimental model of neuro-inflammation, during the chronic phase of the disease. The inventors believe that these myeloid progenitors are recruited into the CNS compartment, providing the CNS with an expandable source of macrophages, dendritic cells and microglia.

On this basis, the invention provides an in vitro or ex vivo method for diagnosis or prognosis of a CNS disease. Such method comprises detecting and/or quantifying CD34+ CD14+ or CD34+ CD11 b+ myeloid progenitor cells in a blood sample of a test subject, wherein an increase of CD34+ CD14+ or CD34+ CD11 b+ myeloid progenitor cells in blood, compared to a reference value, is indicative of a CNS disease.

### Brief description of the drawings:

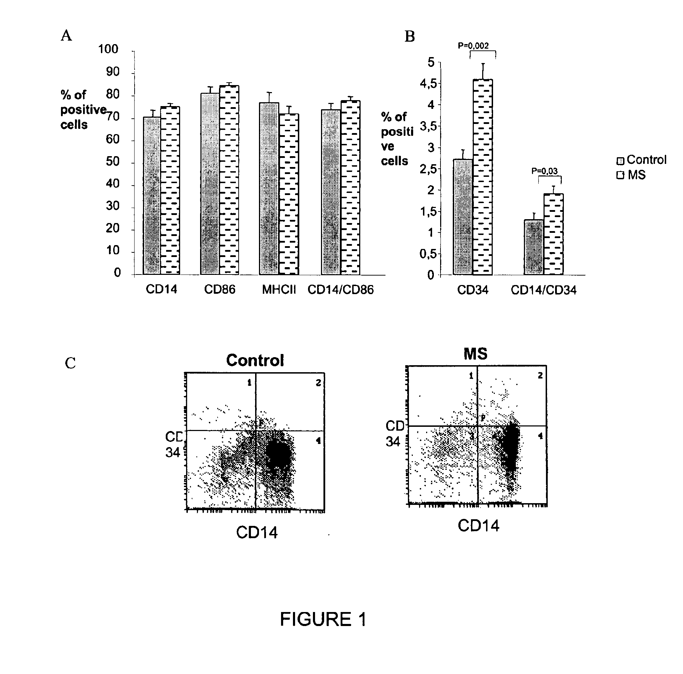

Figure 1 shows a FACS analysis of blood myeloid cells obtained from healthy control subjects or from multiple sclerosis (MS) patients. Peripheral blood mononuclear cells (PBMCs) extracted on Ficoll gradient were obtained from 15 relapsing-remitting MS (RRMS), 15 primary progressive MS (PPMS) and 15 healthy control subjects. Panels A and B are histograms and Panel C is a representative dot blot obtained from a PPMS patient.

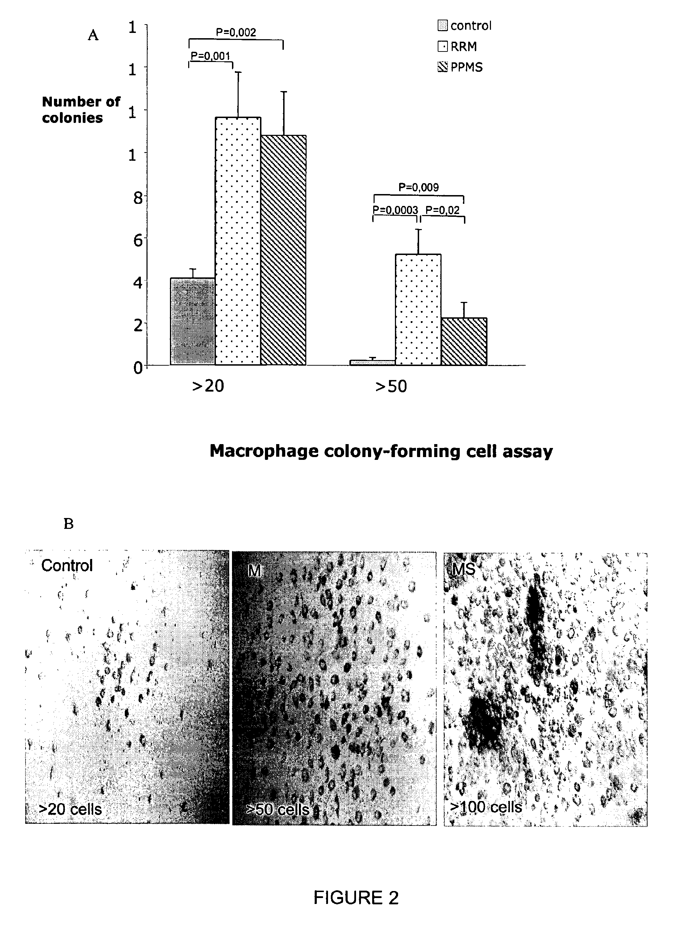

Figure 2 shows a colony-forming cell assay by stimulating PBMC with M-CSF and counting the macrophagic colonies (>20 or >50 cells/colony) developing in semi-solid medium. A significantly higher number of colonies formed in samples deriving from RRMS (n = 5) or PPMS (n = 5) as compared to controls (n = 5) (Fig. 2A). In addition, large colonies (>100 cells/colony) could be observed in MS patients (2 out of 10 patients) but not controls (Fig. 2B).

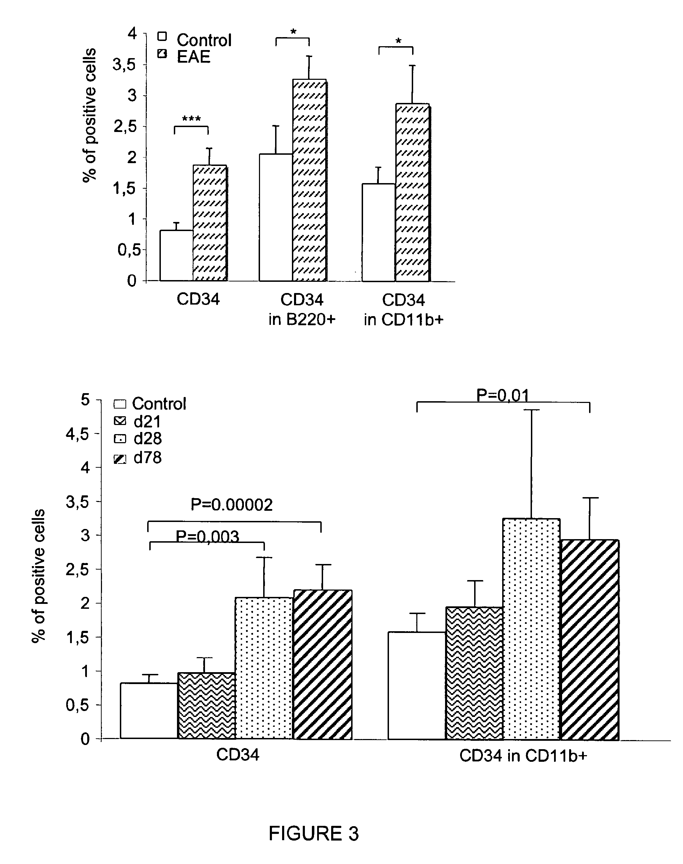

Figure 3 shows a FACS analysis of blood myeloid cells obtained from healthy control mice or from mice suffering from experimental allergic encephalomyelitis, an animal model for multiple sclerosis. Results show that increase of CD34+ CD11 b+ myeloid cells occurs in the chronic phase of the disease, during which the severity of CNS lesions are more pronounced.

### Detailed description of the invention:

### Definitions

The "test subject" is preferably a human, but includes any mammal, (e.g. horses, cats, dogs, cattle, sheep, etc.). The patient may be asymptomatic or may show early or advanced symptoms of a CNS disease.

The "diagnosis" means the identification of the disease or the assessment of the severity of the disease.

The term "prognosis" means the assessment of the outcome of the condition, i.e. to determine the evolution of the condition, and the risk of worsening.

The term "reference" generally refers to a control subject who is either healthy or is a subject who is not affected with a CNS disease but who may be optionally affected with a different condition. It may also be a statistic control value.

In order to determine the evolution of the CNS disease, it may further be very useful to test a patient for the quantity of CD34+ CD14+ or CD34+ CD11 b+ myeloid progenitor cells, and to monitor the effect of a drug or the evolution of the condition, by testing him/her a second time, e. g. a few days or months later. In that case the results of the second test are compared with the results of the first test, and in general also with the results obtained with a "healthy" subject, i.e. a subject who is not affected with a CNS disease. The "reference" then refers either to the same test patient or to a "healthy subject".

"An increase of CD34+ CD14+ or CD34+ CD11 b+ myeloid progenitor cells", in the patient or test subject, compared to a reference value, means that the number of such cells in the blood of the patient is significantly increased versus the reference value. The increase can be e.g. at least 2.5%, or at least 5%, or at least 10%, or at least 20%, more preferably at least 30%.

The "level of activity of the CNS disease" refers to the status of the disease, i.e. to the progression of the disease, and/or to inflammatory episodes of the CNS.

The term "treatment" means prophylactic or curative treatment, including alleviation of symptoms, reduction of number of inflammatory episodes, and/or improvement of the outcome.

The CNS disease may be any disease that affects the central nervous system and more particularly a disease which involves inflammatory mechanisms in the central nervous system. The CNS diseases include more specifically neuroinflammatory or neuro-infectious diseases but also brain tumors and neurodegenerative diseases. Examples of neuroinflammatory diseases include conditions involving demyelinization, such as multiple sclerosis for example, as well as viral or fungal encephalitis, viral or fungal meningitis, tropical spastic paraparesis, HTLV-associated myelopathy, stroke, brain trauma or spinal cord trauma. Mutliple sclerosis is preferred.

Neurodegenerative diseases include Alzheimer's disease, Pick's disease, amyotrophic lateral sclerosis, subacute sclerosing panencephalitis, Huntington's disease, Parkinson's disease, Tay-Sach's disease, Sandhoff disease, multifocal leukoencephalopathy, and prion diseases. Alzheimer's disease is preferred.

The brain tumors may be any tumor that grows in the brain, including, but not limited to, glioma, glioblastoma, medulloblastoma, astrocytoma, and other primitive neuroectoderma. Gliobastoma is preferred.

CNS diseases which involve microglia are preferred.
*CD34+ myeloid progenitor cells as a marker of a CNS inflammatory disease*

The myeloid progenitor cells identified in the invention are CD34+ cells that co-express CD14 and/or CD11b. Their morphology, along with their expression of CD34, may be a further tool of identification, as assessed by FACS (flow cytometry) analysis, based on standard criterion of cell size and granularity allowing monocytes and other mononuclear myeloid cells to be gated.

According to the invention, CD34+ CD14+ or CD34+ CD11 b+ myeloid progenitor cells detected in the blood can be used as a marker of a CNS disease.

The blood sample may consist in whole blood.

Peripheral blood is preferred, and mononuclear cells (PBMCs) are the preferred cells from which CD34+ CD14+ or CD34+ CD11 b+ myeloid progenitor cells can be detected and/or quantified. In a preferred embodiment, the method for diagnosis or prognosis of a CNS disease comprises a preliminary step of extracting peripheral blood mononuclear cells (PBMCs).

An increase of CD34+ myeloid progenitor cells in blood, compared to a reference value, is indicative of a CNS disease.

When the test subject is a patient already diagnosed with a CNS disease, the quantity of CD34+ CD14+ or CD34+ CD11 b+ myeloid progenitor cells in blood of said patient, compared to a former value in the same patient, is indicative of the level of activity of the CNS disease. It can be useful to determine the efficacy of a therapy, by monitoring the progression of the disease in the patient subjected to a defined therapy. The method of the invention is also particularly useful to assess the status of a patient diagnosed with multiple sclerosis.

The detection of CD34+ CD14+ or CD34+ CD11 b+ myeloid progenitor cells can be achieved by flow cytometry (FACS) based on the binding of these cells to anti-CD34 antibodies, and anti-CD14 or anti-CD11b antibodies and optionally on standard morphological criterion allowing myeloid cells to be identified.

Quantification of these cells can be direct numeration, or indirect assessment, e.g. by determining the number of macrophage colonies resulting from the in vitro proliferation and maturation of the CD34+ CD14+ or CD34+ CD11 b+ myeloid progenitor cells stimulated by macrophage-colony stimulating factor (M-CSF). Indeed, when expanded in vitro with M-CSF, such progenitors give rise to macrophagic colonies, which display a specific molecular profile as compared to macrophages derived from controls.

The below examples illustrate the invention without limiting its scope.

### EXEMPLE 1:

Peripheral blood mononuclear cells (PBMCs) extracted on Ficoll gradient were obtained from 15 relapsing-remitting MS (RRMS), 15 primary progressive MS (PPMS) and 15 healthy control subjects. Blood myeloid cells were then assessed by FACS analysis (Figure 1) for the expression of CD14, CD34, MHC class 11 molecules, and the co-stimulatory molecule CD86. The percentage of CD14+ cells, MHC class II+ cells or CD86+ cells was unchanged in MS patients as compared to controls (panel A). In contrast, both RRMS patients and RPMS patients showed increased percentages of CD34+ myeloid cells (4.59+0.5% in RRMS patients, 4.57±0.52% in RPMS patients, 2.71±0.23% in controls, *** p<0.005, Student's t test) (panel B). Such an increase was partly due to the specific expansion of CD14+/CD34+cells as shown by double-staining experiments (see histogram in panel B and a representative dot plot obtained from a PPMS patient in panel C). The inventors then performed a colony-forming cell assay by stimulating PBMC with M-CSF and counting the macrophagic colonies (>20 or >50 cells/colony) developing in semi-solid medium. The inventors observed that a significantly higher number of colonies formed in samples deriving from RRMS (n = 5) or PPMS (n = 5) as compared to controls (n = 5) (Fig. 2A). In addition, large colonies (>100 cells/colony) could be observed in MS patients (2 out of 10 patients) but not controls (Fig. 2B). Finally, preliminary data indicate that such macrophage colonies not only differ quantitatively between MS and controls but also qualitatively, as assessed by immunocytochemical or gene array analyses.

### EXEMPLE 2:

Peripheral blood mononuclear cells (PBMCs) extracted on Ficoll gradient were obtained from 10 control healthy mice and 10 mice suffering from experimental allergic encephalomyelitis (EAE), which were sampled at different time points of the disease. Blood leukocytes were then assessed by FACS analysis for the expression of CD34, CD11b (a marker of myeloid cells) and B220 (a marker found on B-cell and on a sub-population of myeloid cells). In EAE mice as compared to controls, the inventors observed an overall increase of CD34+ cells as well as of CD34+ cells in B220+ cells and of CD34+ cells in CD11 b+ cells (see Figure 3, upper panel). A kinetic analysis showed that such an increase was not statistically significant during the acute phase of the disease (d21) but reached significance during the chronic stage of the disease (from day 28 on), a frame of time characterized by the extension of CNS lesions in EAE mice (see Figure 3, lower panel).

### REFERENCES

Ferrero E, Bondanza A, Leone BE, Manici S, Poggi A, Zocchi MR. CD14+ CD34+ peripheral blood mononuclear cells migrate across endothelium and give rise to immunostimulatory dendritic cells. J Immunol 1998;160:2675-2683
Tabatabai G, Bahr O, Mohle R, et al. Lessons from the bone marrow: how malignant glioma cells attract adult haematopoietic progenitor cells. Brain 2005;128:2200-2211
Tjonnfjord GE, Steen R, Veiby OP, Egeland T. Lineage commitment of CD34+ human hematopoietic progenitor cells. Exp Hematol 1996;24:875-882

## Claims

1. An in vitro method for diagnosis or prognosis of a disease of the central nervous system (CNS), which method comprises detecting and/or quantifying CD34+ CD14+ or CD34+ CD11 b+ myeloid progenitor cells in a blood sample of a test subject, wherein an increase of CD34+ CD14+ or CD34+ CD11 b+ myeloid progenitor cells in blood, compared to a reference value, is indicative of a CNS disease.

2. The method of claim 1, wherein the CNS disease is selected from the group consisting of a neuroinflammatory disease, a neurodegenerative disease and a brain tumor.

3. The method of claim 1 or 2, wherein the CNS disease is multiple sclerosis.

4. The method of claim 1 or 2, wherein the CNS disease is Alzeihmer's disease.

5. The method of claim 1 or 2, wherein the CNS disease is glioma or gliobastoma.

6. The method according to any of claims 1 to 5, wherein the blood sample consists in whole blood or a preparation of peripheral blood mononuclear cells (PBMCs).

7. The method according to any of claims 1 to 6, wherein the CD34+ CD14+ or CD34+ CD11b+ myeloid progenitor cells are quantified by flow cytometry.

8. The method of claim 1, wherein the test subject is a patient already diagnosed with a CNS disease, and the quantity of CD34+ CD14+ or CD34+ CD11 b+ myeloid progenitor cells in blood of said patient, compared to a former value in the same patient, is indicative of the severity or level of activity of the CNS disease.

9. The method of claim 8, wherein the CNS disease is multiple sclerosis.

10. The method of claim 8 or 9, for determining the efficacy of a therapy in said patient.
